(19) Europäisches Patentamt
European Patent Office
Office européen des brevets

(11) EP 2 813 577 A1

(12) **EUROPEAN PATENT APPLICATION**
published in accordance with Art. 153(4) EPC

(43) Date of publication:
**17.12.2014 Bulletin 2014/51**

(21) Application number: **13746840.1**

(22) Date of filing: **06.02.2013**

(51) Int Cl.:
***C12Q 1/28*** *(2006.01)* ***C12Q 1/37*** *(2006.01)*

(86) International application number:
**PCT/JP2013/052669**

(87) International publication number:
**WO 2013/118743 (15.08.2013 Gazette 2013/33)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **09.02.2012 JP 2012026215**

(71) Applicant: **Kyowa Medex Co., Ltd.**
**Tokyo 104-6004 (JP)**

(72) Inventor: **SOYA, Haruyo**
**Sunto-gun**
**Shizuoka 411-0932 (JP)**

(74) Representative: **Vossius & Partner**
**Siebertstrasse 4**
**81675 München (DE)**

(54) **METHOD FOR SUPPRESSING THE EFFECTS OF ASCORBIC ACID**

(57) Provided is a method for suppressing an effect of ascorbic acid on a measurement of glycated hemoglobin in a glycated hemoglobin-containing sample. The method of the present invention is a method for suppressing an effect of ascorbic acid on the measurement of glycated hemoglobin in a glycated hemoglobin-containing sample, comprising reacting the glycated hemoglobin-containing sample with a proteolytic enzyme, reacting a generated glycated peptide with a glycated peptide oxidase, and then measuring a generated hydrogen peroxide, wherein the reaction of the glycated peptide with the glycated peptide oxidase is performed in the presence of a halogen oxide and a substance selected from the group consisting of a substance represented by formula (I), wherein $R^1$ represents substituted or unsubstituted alkyl and a substance represented by the general formula (II), wherein $R^2$ represents substituted or unsubstituted alkyl; andrepresents a single bond or a double bond.

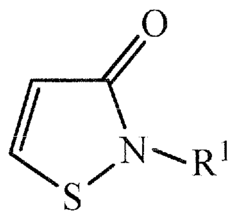

(I)

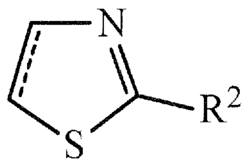

(II)

EP 2 813 577 A1

Printed by Jouve, 75001 PARIS (FR)

## Description

### Technical Field

The present invention relates to a method for suppressing an effect of ascorbic acid on the measurement of glycated hemoglobin, and a reagent for measuring glycated hemoglobin.

### Background Art

Glycated hemoglobin is a glycation product of hemoglobin in which glucose is bound therewith. Hemoglobin takes a tetrameric structure consisting of α and β chains. Its glycation product of the β chain at the N terminus is called hemoglobin A1c, which increases with increase in blood glucose level and as such, is measured as a diabetes mellitus marker in clinical laboratory examinations.

Known methods for measuring glycated hemoglobin include chromatography such as HPLC, electrophoresis, antibody-based immunoassay such as latex immunoagglutination, and enzymatic assay using an enzyme acting on a glycated protein and an enzyme acting on a glycated peptide and/or a glycated amino acid.

A known method for enzymatically measuring glycated hemoglobin (absorptiometry) involves: first denaturing hemoglobin in a hemoglobin-containing sample using a denaturant; reacting a proteolytic enzyme with the denatured hemoglobin; subsequently reacting glycated peptide oxidase with the generated glycated peptide; reacting a generated hydrogen peroxide with a chromogen capable of developing color by oxidation in the presence of a peroxidatively active substance such as peroxidase to convert the chromogen to a dye; and measuring the glycated hemoglobin on the basis of the absorbance of the generated dye.

This measurement of glycated hemoglobin based on absorptiometry is disadvantageously susceptible to an effect of ascorbic acid present in the sample. The conventional measurement of an analyte in a sample using a hydrogen peroxide measurement system is disadvantageously susceptible to the effect of ascorbic acid. To solve a problem to suppress the effect of ascorbic acid, a method using an ascorbic acid oxidase (see patent document 1), a method using an organic complex of iron, cobalt, selenium, copper, mercury, nickel, or the like (see patent documents 2 to 4), a method using iodate (see patent document 5), and a method using iodate in combination with at least one compound selected from a phosphoric acid compound, a boric acid compound, a citric acid compound, a malic acid compound, a tartaric acid compound, and an oxalic acid compound as an adjuvant to the iodate (patent document 6) have been reported. Nonetheless, these methods are insufficiently effective for suppressing the effect of ascorbic acid on the measurement of glycated hemoglobin.

Prior **Art** Documents

Patent Documents

Patent Document 1: Japanese Patent Publication No. 56-39198

Patent Document 2: Japanese Patent Publication No. 1-41223

Patent Document 3: Japanese Patent Publication No. 63-67139

Patent Document 4: Japanese Patent Publication No. 63-39871

Patent Document 5: Japanese unexamined Patent Application Publication No. 56-151358

Patent Document 6: Japanese unexamined Patent Application Publication No. 8-271504

### Summary of the Invention

### Problems to be Solved by the Invention

An object of the present invention is to provide a method for suppressing an effect of ascorbic acid on a measurement of glycated hemoglobin in a glycated hemoglobin-containing sample, and a reagent for measuring glycated hemoglobin.

**Means to Solve the Problems**

The present inventors have conducted diligent studies on the present object and consequently found that in a method for measuring glycated hemoglobin in a glycated hemoglobin-containing sample, an effect of ascorbic acid in the sample can be suppressed by reacting a proteolytic enzyme with the glycated hemoglobin-containing sample, then reacting a generated glycated peptide with a glycated peptide oxidase, and measuring a generated hydrogen peroxide, wherein the reaction of the glycated peptide with the glycated peptide oxidase is performed in the presence of a halogen oxide and a specific 5-membered heterocyclic compound comprising a nitrogen atom and a sulfur atom. On the basis of these findings, the present invention has been completed. Specifically, the present invention relates to the following [1] to [7]:

[1] A method for suppressing an effect of ascorbic acid on a measurement of glycated hemoglobin in a glycated hemoglobin-containing sample, comprising reacting a proteolytic enzyme with the glycated hemoglobin-containing sample, then reacting a generated glycated peptide with a glycated peptide oxidase, and measuring a generated hydrogen peroxide, wherein the reaction of the glycated peptide with the glycated peptide oxidase is performed in the presence of a halogen oxide and a substance selected from the group consisting of a substance represented by formula (I):

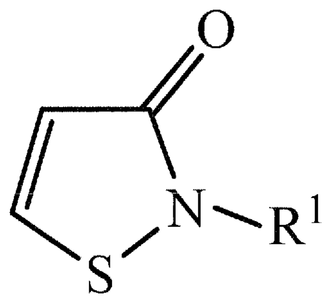

(I)

wherein $R^1$ represents substituted or unsubstituted alkyl, and a substance represented by the general formula (II):

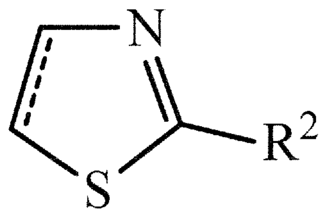

(II)

wherein $R^2$ represents substituted or unsubstituted alkyl; and --- represents a single bond or a double bond.
[2] The method according to [1], wherein the halogen oxide is a halogen oxide selected from the group consisting of iodic acid or a salt thereof and bromic acid or a salt thereof.
[3] The method according to [1] or [2], wherein the glycated hemoglobin is hemoglobin A1c.
[4] A reagent for measuring glycated hemoglobin in a sample, comprising a proteolytic enzyme, a glycated peptide oxidase, a halogen oxide, a substance selected from the group consisting of a substance represented by the general formula (I):

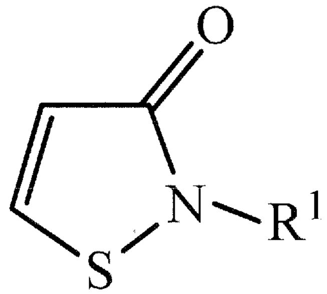

(I)

wherein $R^1$ represents substituted or unsubstituted alkyl, and a substance represented by the general formula (II):

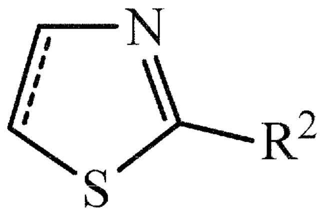

(II)

wherein $R^2$ represents substituted or unsubstituted alkyl; and --- represents a single bond or a double bond, and a reagent for measuring hydrogen peroxide.

[5] The reagent according to [4], wherein the halogen oxide is a halogen oxide selected from the group consisting of iodic acid or a salt thereof and bromic acid or a salt thereof.

[6] The reagent according to [4] or [5], wherein the glycated hemoglobin is hemoglobin A1c.

[7] The reagent according to any one of [4] to [6], wherein the reagent is intended to suppress the effect of ascorbic acid on the measurement of glycated hemoglobin in the sample.

**Effect of the Invention**

The present invention provides a method for suppressing an effect of ascorbic acid on the measurement of glycated hemoglobin, and a reagent for measuring glycated hemoglobin.

**Mode of Carrying Out the Invention**

(1) Method for suppressing an effect of ascorbic acid on a measurement of glycated hemoglobin in a glycated hemoglobin-containing sample

The method for suppressing an effect of ascorbic acid on a measurement of glycated hemoglobin in a glycated hemoglobin-containing sample according to the present invention is a method for suppressing the effect of ascorbic acid on a measurement of glycated hemoglobin in a glycated hemoglobin-containing sample, comprising reacting a proteolytic enzyme with the glycated hemoglobin-containing sample, then reacting a generated glycated peptide with a glycated peptide oxidase, and measuring a generated hydrogen peroxide, wherein the reaction of the glycated peptide with the glycated peptide oxidase is performed in the presence of a halogen oxide and a substance selected from the group consisting of a substance represented by formula (I):

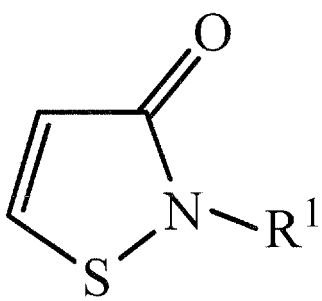

(I)

wherein $R^1$ represents substituted or unsubstituted alkyl, [hereinafter, referred to as compound (I)] and a substance represented by formula (II):

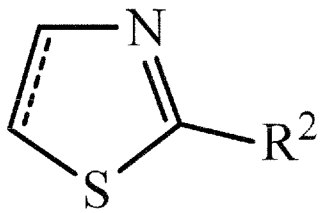

(II)

wherein $R^2$ represents substituted or unsubstituted alkyl; and --- represents a single bond or a double bond [hereinafter, referred to as compound (II)]. The halogen oxide and the substance selected from the group consisting of compound (I) and compound (II) can coexist in the reaction of the glycated hemoglobin-containing sample with the proteolytic enzyme.

The glycated hemoglobin-containing sample in the measurement method of the present invention is not particularly limited as long as the sample contains glycated hemoglobin and hemoglobin and enables the method for suppressing an effect of ascorbic acid according to the present invention. Examples thereof include whole blood, blood cells, mixed samples of blood cells and plasma, and hemolyzed samples of these samples. The hemolyzing treatment is not particularly limited as long as the treatment hemolyzes whole blood, blood cells, or mixed samples of blood cells and plasma. Examples thereof include a physical method, a chemical method, and a biological method. Examples of the physical method include a method using a hypotonic solution such as distilled water, and a method using sonic waves. Examples of the chemical method include a method using an organic solvent such as methanol, ethanol, or acetone, and a method using a polyoxyethylene surfactant. Examples of the biological method include a method using an antibody or a complement.

The glycated hemoglobin according to the present invention is generated by the binding of a sugar such as glucose to hemoglobin. Examples thereof include hemoglobin A1a, hemoglobin A1b, and hemoglobin A1c, and is preferred hemoglobin A1c.

The halogen oxide according to the present invention is not particularly limited as long as the halogen oxide enables the method for suppressing an effect of ascorbic acid according to the present invention. Examples thereof include iodic acid or a salt thereof, bromic acid or a salt thereof, and periodic acid or a salt thereof. Iodic acid or a salt thereof, or bromic acid or a salt thereof is preferred. Examples of the salts include lithium salt, sodium salt, potassium salt, ammonium salt, calcium salt, and magnesium salt.

Specific examples (products) of the halogen oxide include iodic acid, sodium iodate, potassium iodate, bromic acid, sodium bromate, potassium bromate, periodic acid, sodium periodate, and potassium periodate.

In the method for measuring glycated hemoglobin according to the present invention, the concentration of the halogen oxide in the reaction solution is not particularly limited as long as the concentration enables the method for measuring glycated hemoglobin according to the present invention. The concentration is usually 0.005 to 20 mmol/L, preferably 0.01 to 10 mmol/L.

$R^1$ in the compound (I) according to the present invention represents a substituted or unsubstituted alkyl. Examples of the alkyl in the substituted or unsubstituted alkyl include a linear alkyl having 1 to 20 carbon atoms and a branched alkyl having 3 to 20 carbon atoms. Examples of the linear alkyl having 1 to 20 carbon atoms include methyl, ethyl, propyl, butyl, pentyl, hexyl, heptyl, octyl, nonyl, decyl, undecyl, dodecyl (lauryl), tridecyl, tetradecyl (myristyl), pentadecyl, hexadecyl (cetyl), heptadecyl, octadecyl (stearyl), nonadecyl, and icosyl. Examples of the branched alkyl having 3 to 20 carbon atoms include isopropyl, isobutyl, isopentyl, isohexyl, isoheptyl, isooctyl, isononyl, isodecyl, isoundecyl,, isododecyl, isotridecyl, isotetradecyl, isopentadecyl, isohexadecyl, isoheptadecyl, isooctadecyl, isononadecyl, isoicosyl, and octyldodecyl. Examples of the substituent(s) in the substituted alkyl include a phenyl group, a hydroxy group, a sulfo group, a cyano group, and a halogen atom. Examples of the halogen atom include a chlorine atom, a bromine atom, and an iodine atom.

Specific examples (products) of the compound (I) include 2-octyl-4-isothiazolin-3-one (manufactured by Tokyo Chemical Industry Co., Ltd. and manufactured by CHEMICREA Inc.) and 2-methyl-4-isothiazolin-3-one (manufactured by Sigma-Aldrich Corp.).

$R^2$ in the compound (II) according to the present invention represents a substituted or unsubstituted alkyl. Examples of the alkyl in the substituted or unsubstituted alkyl include a linear alkyl having 1 to 20 carbon atoms and a branched alkyl having 3 to 20 carbon atoms. Examples of the linear alkyl having 1 to 20 carbon atoms include methyl, ethyl, propyl, butyl, pentyl, hexyl, heptyl, octyl, nonyl, decyl, undecyl, dodecyl (lauryl), tridecyl, tetradecyl (myristyl), pentadecyl, hexadecyl (cetyl), heptadecyl, octadecyl (stearyl), nonadecyl, and icosyl. Examples of the branched alkyl having 3 to 20 carbon atoms include isopropyl, isobutyl, isopentyl, isohexyl, isoheptyl, isooctyl, isononyl, isodecyl, isoundecyl, isododecyl, isotridecyl, isotetradecyl, isopentadecyl, isohexadecyl, isoheptadecyl, isooctadecyl, isononadecyl, isoicosyl, and octyldodecyl. Examples of the substituent(s) in the substituted alkyl include a phenyl group, a hydroxy group, a sulfo group, a cyano group, and a halogen atom. Examples of the halogen atom include a chlorine atom, a bromine atom, and an iodine atom.

Specific examples (products) of the compound (II) include 2-methylthiazoline (manufactured by Tokyo Chemical Industry Co., Ltd.), 2-methylthiazole (manufactured by Tokyo Chemical Industry Co., Ltd.), 2-ethylthiazole (manufactured by Tokyo Chemical Industry Co., Ltd.), 2-propylthiazole (manufactured by Tokyo Chemical Industry Co., Ltd.), and 2-isobutylthiazole (manufactured by Tokyo Chemical Industry Co., Ltd.).

In the method for suppressing an effect of ascorbic acid according to the present invention, the concentration of the compound (I) or the compound (II) in the reaction solution is not particularly limited as long as the concentration enables the method for measuring glycated hemoglobin according to the present invention. The concentration is usually

0.005 to 20 mmol/L, preferably 0.01 to 10 mmol/L.

The reaction of a proteolytic enzyme with the glycated hemoglobin-containing sample to generate a glycated peptide can be performed under any condition as long as a glycated peptide can be generated. This reaction, i.e., the reaction of glycated hemoglobin in the glycated hemoglobin-containing sample with the proteolytic enzyme, is preferably performed in an aqueous medium. Examples of the aqueous medium include an aqueous medium mentioned later. This reaction can also be performed in the presence of a denaturant for the glycated hemoglobin in the glycated hemoglobin-containing sample. Since this denaturant renders the glycated hemoglobin reactable with the proteolytic enzyme, the reaction of the glycated hemoglobin with the proteolytic enzyme is preferably performed in the presence of the denaturant. The denaturant is not particularly limited as long as the denaturant can render the glycated hemoglobin reactable with the proteolytic enzyme. Examples thereof include a cationic surfactant, an anionic surfactant, an amphoteric surfactant, and a nonionic surfactant.

Examples of the cationic surfactants include a quaternary ammonium salt and a pyridinium salt.

The quaternary ammonium salt is preferably a quaternary ammonium salt having at least one linear alkyl having 8 to 20 carbon atoms. Examples of the linear alkyl having 8 to 20 carbon atoms include octyl, nonyl, decyl, undecyl, dodecyl (lauryl), tridecyl, tetradecyl (myristyl), pentadecyl, hexadecyl (cetyl), heptadecyl, octadecyl (stearyl), nonadecyl, and icosyl.

Specific examples (products) of the quaternary ammonium salt include decyltrimethylammonium chloride, decyltrimethylammonium bromide, dodecyltrimethylammonium chloride, dodecyltrimethylammonium bromide, tetradecyltrimethylammonium bromide (hereinafter, referred to as C14TMA), hexadecyltrimethylammonium chloride, hexadecyltrimethylammonium bromide, didecyldimethylammonium chloride, didecyldimethylammonium bromide, didodecyldimethylammonium chloride, and didodecyldimethylammonium bromide (all manufactured by Tokyo Chemical Industry Co., Ltd.).

Examples of the pyridinium salt include an N-alkylpyridinium salt and an N-alkenylpyridinium salt. Examples of the alkyl include linear alkyl having 1 to 20 carbon atoms and branched alkyl having 3 to 20 carbon atoms. Examples of the linear alkyl having 1 to 20 carbon atoms include methyl, ethyl, propyl, butyl, pentyl, hexyl, heptyl, octyl, nonyl, decyl, undecyl, dodecyl (lauryl), tridecyl, tetradecyl (myristyl), pentadecyl, hexadecyl (cetyl), heptadecyl, octadecyl (stearyl), nonadecyl, and icosyl. Examples of the branched alkyl having 3 to 20 carbon atoms include isopropyl, isobutyl, isopentyl, isohexyl, isoheptyl, isooctyl, isononyl, isodecyl, isoundecyl, isododecyl, isotridecyl, isotetradecyl, isopentadecyl, isohexadecyl, isoheptadecyl, isooctadecyl, isononadecyl, isoicosyl, and octyldodecyl.

Examples of the alkenyl include alkenyl having 2 to 20 carbon atoms. Examples of the alkenyl having 2 to 20 carbon atoms include vinyl, propenyl, allyl, butenyl, pentenyl, hexenyl, heptenyl, octenyl, nonenyl, decenyl, undecenyl, dodecenyl, tetradecenyl, pentadecenyl, hexadecenyl, heptadecenyl, octadecenyl, oleyl, nonadecenyl, and icosenyl.

Alternatively, a substituted alkyl can also be used as the alkyl, and a substituted alkenyl can also be used as the alkenyl. Examples of the substituent(s) in the substituted alkyl or the substituted alkenyl include a phenyl group, a hydroxy group, a sulfo group, a cyano group, and a halogen atom. Examples of the halogen atom include a chlorine atom, a bromine atom, and an iodine atom.

Alternatively, an N-alkylpyridinium salt or an N-alkenylpyridinium salt each having substituent(s) at positions 2 to 6 on the pyridine ring can also be used. Examples of the substituent(s) at positions 2 to 6 on the pyridine ring include a linear alkyl having 1 to 20 carbon atoms, a branched alkyl having 3 to 20 carbon atoms, and an alkenyl having 2 to 20 carbon atoms. Examples of the linear alkyl having 1 to 20 carbon atoms include the aforementioned linear alkyl having 1 to 20 carbon atoms. Examples of the branched alkyl having 3 to 20 carbon atoms include the aforementioned branched alkyl having 3 to 20 carbon atoms. Examples of the alkenyl having 2 to 20 carbon atoms include the aforementioned linear alkenyl having 2 to 20 carbon atoms.

Specific examples (products) of the pyridinium salt include 1-dodecylpyridinium chloride (hereinafter, referred to as C12py; manufactured by Tokyo Chemical Industry Co., Ltd.), 1-cetylpyridinium chloride (manufactured by Tokyo Chemical Industry Co., Ltd.), 1-cetyl-4-methylpyridinium chloride (manufactured by Tokyo Chemical Industry Co., Ltd.), and N-octadecyl-4-stilbazole bromide (manufactured by Tokyo Chemical Industry Co., Ltd.).

Examples of the anionic surfactant include a sulfuric acid ester salt, a carboxylate, a sulfonate, a phosphoric acid ester salt, a sulfosuccinate, an N-methyltaurine salt, and an N-alkanoyl-N-methyltaurine salt.

Examples of the amphoteric surfactant include a tertiary amine oxide and an alkylcarboxybetaine.

Examples of the nonionic surfactant include a polycxyethylene alkylamine, a polyoxyethylene alkenylamine, a polyoxyethylene alkyl ether, a polyoxyethylene alkenyl ether, a polyoxyethylene alkylphenyl ether, an ethylenediamine tetrapolyoxyethylene, and a polyglycerin fatty acid ester, and is preferred a polyoxyethylene alkylamine or a polyoxyethylene alkyl ether.

Examples of the alkyl in the polyoxyethylene alkylamine include an alkyl having 8 to 20 carbon atoms. Examples of the alkyl having 8 to 20 carbon atoms include octyl, nonyl, decyl, undecyl, dodecyl (lauryl), tridecyl, tetradecyl (myristyl), pentadecyl, hexadecyl (cetyl), heptadecyl, octadecyl (stearyl), nonadecyl, and icosyl.

Examples of the alkenyl in the polyoxyethylene alkenylamine include an alkenyl having 8 to 20 carbon atoms.

Examples of the alkenyl having 8 to 20 carbon atoms include octenyl, nonenyl, decenyl, undecenyl, dodecenyl, tetradecenyl, pentadecenyl, hexadecenyl, heptadecenyl, octadecenyl, oleyl, nonadecenyl, and icosenyl.

Examples of the alkyl in the polyoxyethylene alkyl ether include an alkyl having 8 to 20 carbon atoms. Examples of the alkyl having 8 to 20 carbon atoms include the aforementioned alkyl having 8 to 20 carbon atoms.

Examples of the alkenyl in the polyoxyethylene alkenyl ether include an alkenyl having 8 to 20 carbon atoms. Examples of the alkenyl having 8 to 20 carbon atoms include the aforementioned alkenyl having 8 to 20 carbon atoms.

Examples of the alkyl in the polyoxyethylene alkylphenyl ether include an alkyl having 8 to 20 carbon atoms. Examples of the alkyl having 8 to 20 carbon atoms include the aforementioned alkyl having 8 to 20 carbon atoms.

The reaction of the glycated hemoglobin in the glycated hemoglobin-containing sample with the proteolytic enzyme is performed usually at 10 to 50°C, preferably 20 to 40°C, and usually for 1 minute to 3 hours, preferably 2.5 minutes to 1 hour. The concentration of the proteolytic enzyme is not particularly limited as long as the reaction of the glycated hemoglobin in the hemoglobin-containing sample with the proteolytic enzyme can proceed. The concentration is usually 50 to 25,000 kU/L, preferably 250 to 10,000 kU/L.

The proteolytic enzyme is not particularly limited as long as the enzyme reacts with a glycated hemoglobin in the hemoglobin-containing sample to generate a glycated peptide from the glycated hemoglobin. Examples thereof include a serine protease (chymotrypsin, subtilisin, etc.), a cysteine protease (papain, caspase, etc.), an aspartic acid protease (pepsin, cathepsin D, etc.), a metalloprotease (thermolysin, etc.), an N-terminal threonine protease, and a glutamic acid protease. In the present invention, a commercially available proteolytic enzyme can also be used. Examples of the commercially available product include Protease P "Amano" 3G and Protease K "Amano" (both manufactured by Amano Enzyme Inc.), Actinase AS and Actinase E (both manufactured by Kaken Pharma Co., Ltd.), Thermolysin (manufactured by Daiwa Fine Chemicals Co., Ltd.), and Sumizyme MP (manufactured by Shin Nihon Chemical Co., Ltd.).

The concentration of the denaturant in the reaction of the proteolytic enzyme is not particularly limited as long as the reaction of the glycated hemoglobin in the glycated hemoglobin-containing sample with the proteolytic enzyme can proceed. The concentration is usually 0.0001 to 10%, preferably 0.0005 to 5%.

The reaction of the glycated hemoglobin in the glycated hemoglobin-containing sample with the proteolytic enzyme generates a glycated peptide. The generated glycated peptide is reacted with a glycated peptide oxidase to generate hydrogen peroxide. The reaction of the glycated peptide with the glycated peptide oxidase is preferably performed in an aqueous medium. Examples of the aqueous medium include an aqueous medium mentioned later.

The reaction of the glycated peptide with the glycated peptide oxidase is performed usually at 10 to 50°C, preferably 20 to 40°C, and usually for 1 minute to 3 hours, preferably 2.5 minutes to 1 hour. The concentration of the glycated peptide oxidase is not particularly limited as long as the reaction of the fructosyl hemoglobin with the glycated peptide oxidase can proceed. The concentration is usually 0.1 to 30 kU/L, preferably 0.2 to 15 kU/L.

The glycated peptide oxidase is not particularly limited as long as the enzyme reacts with the glycated peptide to generate hydrogen peroxide. Examples thereof include glycated peptide oxidases derived from filamentous bacteria, yeasts, actinomycetes, bacteria, or archaebacteria. In the present invention, a commercially available glycated peptide oxidase can also be used. Examples of the commercially available product include FPOX-CE (manufactured by Kikkoman Corp.), FPOX-EE (manufactured by Kikkoman Corp.), and FPOX-CET (manufactured by Kikkoman Corp.).

Examples of the method for measuring the generated hydrogen peroxide include a method using an electrode, and a method using a reagent for measuring hydrogen peroxide. A method using a reagent for measuring hydrogen peroxide is preferred. The reagent for measuring hydrogen peroxide refers to a reagent for converting hydrogen peroxide to a detectable substance. Examples of the detectable substance include a dye, a light (luminescence), and a fluorescence, and is preferred a dye.

In case the detectable substance is a dye, examples of the reagent for measuring hydrogen peroxide include a reagent comprising a peroxidatively active substance such as peroxidase and a chromogen capable of developing color by oxidation. Examples of the chromogen capable of developing color by oxidation include an oxidative coupling-type chromogen and a leuco chromogen, and is preferred a leuco chromogen. Examples of the leuco chromogen include a phenothiazine chromogen, a triphenylmethane chromogen, a diphenylamine chromogen, o-phenylenediamine, hydroxypropionic acid, diaminobenzidine, and tetramethylbenzidine, and is preferred a phenothiazine chromogen. Examples of the phenothiazine chromogen include 10-N-carboxymethylcarbamoyl-3,7-bis(dimethylamino)-10H-phenothiazine (CCAP), 10-N-methylcarbamoyl-3,7-bis(dimethylamino)-10H-phenothiazine (MCDP), and 10-N-(carboxymethylaminocarbonyl)-3,7-bis(dimethylamino)-10H-phenothiazine sodium salt (DA-67), and is particularly preferred is 10-N-(carboxymethylaminocarbonyl)-3,7-bis(dimethylamino)-10H-phenothiazine sodium salt (DA-67). Examples of the triphenylmethane chromogen include N,N,N',N',N",N"-hexa(3-sulfopropyl)-4,4',4"-triaminotriphenylmethane (TPM-PS). Examples of the diphenylamine chromogen include N-(carboxymethylaminocarbonyl)-4,4'-bis(dimethylamino)diphenylamine sodium salt (DA-64), 4,4'-bis(dimethylamino)diphenylamine, and bis[3-bis(4-chlorophenyl)methyl-4-dimethylaminophenyl]amine (BCMA).

In case the detectable substance is light (luminescence), examples of the reagent for measuring hydrogen peroxide include a reagent comprising a peroxidatively active substance such as peroxidase and a chemiluminescent

substance. Examples of the chemiluminescent substance include luminol, isoluminol, lucigenin, and an acridinium ester.

In case the detectable substance is fluorescence, examples of the reagent for measuring hydrogen peroxide include a reagent comprising a peroxidatively active substance such as peroxidase and a fluorescent substance. Examples of the fluorescent substance include 4-hydroxyphenylacetic acid, 3-(4-hydroxyphenyl)propionic acid, and coumarin.

The method for measuring glycated hemoglobin in the glycated hemoglobin-containing sample according to the present invention also encompasses a method involving calculating the ratio of the amount of glycated hemoglobin to the amount of total hemoglobin in the glycated hemoglobin-containing sample.

The amount of total hemoglobin can be determined by a method known in the art, for example, a cyanmethemoglobin method, an oxyhemoglobin method, or an SLS-hemoglobin method. The amount of total hemoglobin can also be determined by applying the cyanmethemoglobin method, the oxyhemoglobin method, or the SLS-hemoglobin method not only to the sample itself comprising glycated hemoglobin but to a sample obtained by the addition of the halogen oxide and the substance selected from the group consisting of compound (I) and compound (II) to the glycated hemoglobin-containing sample or a sample obtained by the addition of the halogen oxide, the substance selected from the group consisting of compound (I) and compound (II), and the proteolytic enzyme to the glycated hemoglobin-containing sample.

(2) Reagent for measuring glycated hemoglobin in a glycated hemoglobin-containing sample

The reagent for measuring glycated hemoglobin in a glycated hemoglobin-containing sample according to the present invention is a reagent comprising a proteolytic enzyme, a glycated peptide oxidase, a halogen oxide, a substance selected from the group consisting of compound (I) and compound (II), and a reagent for measuring hydrogen peroxide. The reagent for measuring glycated hemoglobin according to the present invention is used in the method for suppressing an effect of ascorbic acid according to the present invention.

Examples of the proteolytic enzyme, the glycated peptide oxidase, the halogen oxide, the compound (I), the compound (II), and the reagent for measuring hydrogen peroxide in the measurement reagent of the present invention include the aforementioned proteolytic enzyme, glycated peptide oxidase, halogen oxide, compound (I), compound (II), and reagent for measuring hydrogen peroxide, respectively.

A concentration of the proteolytic enzyme in the measurement reagent of the present invention is usually 50 to 25,000 kU/L, preferably 250 to 10,000 kU/L. The concentration of the glycated peptide oxidase in the measurement reagent of the present invention is usually 0.1 to 30 kU/L, preferably 0.2 to 15 kU/L.

A concentration of the halogen oxide in the measurement reagent of the present invention is usually 0.005 to 20 mmol/L, preferably 0.01 to 10 mmol/L.

A concentration of the compound (I) in the measurement reagent of the present invention is usually 0.005 to 20 mmol/L, preferably 0.01 to 10 mmol/L.

A concentration of the compound (II) in the measurement reagent of the present invention is usually 0.005 to 20 mmol/L, preferably 0.01 to 10 mmol/L.

The measurement reagent of the present invention can optionally comprise an aqueous medium, a denaturant, a stabilizer, an antiseptic, salts, an interference inhibitor, a solubilizer, an organic solvent, and the like.

Examples of the aqueous medium include deionized water, distilled water, and buffer solution, and is preferred a buffer solution.

A pH of the aqueous medium is, for example, pH 4 to 10. In case of using a buffer solution as the aqueous medium, a buffer is preferably used according to the set pH. Examples of the buffer used in the buffer solution include a tris(hydroxymethyl)aminomethane buffer, a phosphate buffer, a borate buffer, and a Good's buffer.

Examples of the Good's buffer include 2-morpholinoethanesulfonic acid (MES), bins(2-hydroxyethyl)iminotris(hydroxymethyl)methane (Bis-Tris), N-(2-acetamide)iminodiacetic acid (ADA), piperazine-N,N'-bis(2-ethanesulfonic acid) (PIPES), N-(2-acetamido)-2-aminoethanesulfonic acid (ACES), 3-morpholino-2-hydroxypropanesulfonic acid (MOPSO), N,N-bis(2-hydroxyethyl)-2-aminoethanesulfonic acid (BES), 3-morpholinopropanesulfonic acid (MOPS), N-[tris(hydroxymethyl)methyl]-2-aminoethanesulfonic acid (TES), 2-[4-(2-hydroxyethyl)-1-piperazinyl]ethanesulfonic acid (HEPES), 3-[N,N-bis(2-hydroxyethyl)amino]-2-hydroxypropanesulfonic acid (DIPSO), N-[tris(hydroxymethyl)methyl]-2-hydroxy-3-aminopropanesulfonic acid (TAPSO), piperazine-N,N'-bis(2-hydroxypropanesulfonic acid) (POPSO), 3-[4-(2-hydroxyethyl)-1-piperazinyl]-2-hydroxypropanesulfonic acid (HEPPSO), 3-[4-(2-hydroxyethyl)-1-piperazinyl]propanesulfonic acid [(H)EPPS], N-[tris(hydroxymethyl)methyl]glycine (Tricine), N,N-bis(2-hydroxyethyl)glycine (Bicine), N-tris(hydroxymethyl)methyl-3-aminopropanesulfonic acid (TAPS), N-cyclohexyl-2-aminoethanesulfonic acid (CHES), N-cyclohexyl-3-amino-2-hydroxypropanesulfonic acid (CAPSO), and N-cyclohexyl-3-aminopropanesulfonic acid (CAPS).

A concentration of the buffer solution is usually 0.001 to 2.0 mol/L, preferably 0.005 to 1.0 mol/L.

Examples of the denaturant include the aforementioned denaturant. The concentration of the denaturant in the measurement reagent of the present invention is usually 0.0001 to 10%, preferably 0.0005 to 5%.

Examples of the stabilizer include ethylenediaminetetraacetic acid (EDTA), sucrose, calcium chloride, calcium acetate, calcium nitrate, potassium ferrocyanide, and bovine serum albumin (BSA). Examples of the antiseptic include sodium azide and an antibiotic. Examples of the salt include sodium chloride, sodium nitrate, sodium sulfate, sodium carbonate, potassium chloride, potassium nitrate, potassium sulfate, and potassium carbonate. Examples of the interference inhibitor include pyruvic acid for suppressing an effect of peroxide. Examples of the solubilizer include a nonionic surfactant such as a polyoxyethylene alkyl ether and a polyoxyethylene alkylphenyl ether. Examples of the organic solvent include dimethylformamide (DMF), dimethyl sulfoxide (DMSO), dioxane, acetone, methanol, and ethanol, which make the leuco chromogen soluble in the aqueous medium.

The reagent for measuring glycated hemoglobin according to the present invention can be in the form of a kit. Examples of the kit for measuring glycated hemoglobin include a two-reagent system kit and a three-reagent system kit. In case the kit for measuring glycated hemoglobin is a two-reagent system kit, an arrangement of the components, i.e., the proteolytic enzyme, the glycated peptide oxidase, the halogen oxide, the substance selected from the group consisting of compound (I) and compound (II), and the reagent for measuring hydrogen peroxide, can be appropriately selected by those skilled in the art in view of the preservation stability of the kit. A kit comprising the proteolytic enzyme and the glycated peptide oxidase in separate reagents is preferred.

Hereinafter, the present invention will be described in more detail with reference to Examples. However, the scope of the present invention is not limited to these examples by any means. In the present Examples, reagents and enzymes from the following manufacturers were used:

Bis-Tris (manufactured by Dojindo Laboratories), MOPS (manufactured by Dojindo Laboratories), calcium chloride dihydrate (manufactured by Kanto Chemical Co., Inc.), sodium pyruvate (manufactured by Kanto Chemical Co., Inc.), DMSO (manufactured by Nacalai Tesque, Inc.), DA-67 (manufactured by Wako Pure Chemical Industries, Ltd.), Triton X-405 (nonionic surfactant; manufactured by Sigma-Aldrich Corp.), 1-dodecylpyridinium chloride (C12py) (denaturant; manufactured by Tokyo Chemical Industry Co., Ltd.), tetradecyltrimethylammonium bromide (C14TMA) (denaturant; manufactured by Tokyo Chemical Industry Co., Ltd.), potassium iodate (halogen oxide; manufactured by Tokyo Chemical Industry Co., Ltd.), potassium bromate (halogen oxide; manufactured by Tokyo Chemical Industry Co., Ltd.), 2-octyl-4-isothiazolin-3-one [compound (I); manufactured by CHEMICREA Inc.], ProClin 950 {9.5% aqueous solution of 2-methyl-4-isothiazolin-3-one [compound (I)]; manufactured by Sigma-Aldrich Corp.}, 2-methylthiazoline [compound (II); manufactured by Tokyo Chemical Industry Co., Ltd.], Actinase E (proteolytic enzyme; manufactured by Kaken Pharma Co., Ltd.), FPOX-CET (glycated peptide oxidase; manufactured by Kikkoman Corp.), and peroxidase (manufactured by Toyobo Co., Ltd.).

Example 1

A kit for measuring HbA1c, consisting of the following first and second reagents (kits 1 to 3 and kits a to e), was prepared as shown in Table 1.

| | |
|---|---|
| First reagent | |
| Bis-Tris (pH 6.8) | 10 mmol/L |
| C12py | 1.2 g/L |
| Potassium iodate | |
| Compound (I) or Compound (II) | |
| Calcium chloride dihydrate | 16 mmol/L |
| Sodium pyruvate | 2 g/L |
| Actinase E | 340 kU/L |
| DMSO | 0.2 mL/L |
| DA-67 | 12.6 μmol/L |
| Second reagent | |
| MOPS (pH 7.0) | 50 mmol/L |
| Triton X-405 | 7.1 g/L |
| Peroxidase | 80 kU/L |
| FPOX-CET | 2.5 kU/L |

[Table 1]

Table 1

| Kit | Halogen oxide | Compound (I) or Compound (II) |
|---|---|---|
| Kit 1 | Potassium iodate (2.8 mmol/L) | 2-Octyl-4-isothiazolin-3-one (2.3 mmol/L) |
| Kit 2 | Potassium iodate (2.8 mmol/L) | 2-Methyl-4-isothiazolin-3-one (4.1 mmol/L) |
| Kit 3 | Potassium iodate (2.8 mmol/L) | 2-Methylthiazoline (4.9 mmol/L) |
| Kit a | Potassium iodate (2.8 mmol/L) | - |
| Kit b | - | 2-Octyl-4-isothiazolin-3-one (2.3 mmol/L) |
| Kit c | - | 2-Methyl-4-isothiazolin-3-one (4.1 mmol/L) |
| Kit d | - | 2-Methylthiazoline (4.9 mmol/L) |
| Kit e | - | - |

Example 2

A kit for measuring HbA1c, consisting of the following first and second reagents (kits 4 to 6 and kits f to j), was prepared as shown in Table 2.

| | |
|---|---|
| First reagent | |
| Bis-Tris (pH 6.8) | 10 mmol/L |
| C14TMA | 0.6 g/L |
| Potassium iodate | |
| Compound (I) or Compound (II) | |
| Calcium chloride dihydrate | 16 mmol/L |
| Sodium pyruvate | 2 g/L |
| Actinase E | 340 kU/L |
| DMSO | 0.2 mL/L |
| DA-67 | 12.6 $\mu$mol/L |
| Second reagent | |
| MOPS (pH 7.0) | 50 mmol/L |
| Triton X-405 | 7.1 g/L |
| Peroxidase | 80 kU/L |
| FPOX-CET | 2.5 kU/L |

[Table 2]

| Kit | Halogen oxide | Compound (I) or Compound (II) |
|---|---|---|
| Kit 4 | Potassium iodate (2.8 mmol/L) | 2-Octyl-4-isothiazolin-3-one (2.3 mmol/L) |
| Kit 5 | Potassium iodate (2.8 mmol/L) | 2-Methyl-4-isothiazolin-3-one (4.1 mmol/L) |
| Kit 6 | Potassium iodate (2.8 mmol/L) | 2-Methylthiazoline (4.9 mmol/L) |
| Kit f | Potassium iodate (2.8 mmol/L) | - |
| Kit g | - | 2-Octyl-4-isothiazolin-3-one (2.3 mmol/L) |
| Kit h | - | 2-Methyl-4-isothiazolin-3-one (4.1 mmol/L) |
| Kit i | - | 2-Methylthiazoline (4.9 mmol/L) |
| Kit j | - | - |

Example 3

A kit for measuring HbA1c, consisting of the following first and second reagents (kits 7 and 8 and kits k and g

to j), was prepared as shown in Table 3.

| | |
|---|---|
| First reagent | |
| Bis-Tris (pH 6.8) | 10 mmol/L |
| C14TMA | 0.6 g/L |
| Potassium bromate | |
| Compound (I) or Compound (II) | |
| Calcium chloride dihydrate | 16 mmol/L |
| Sodium pyruvate | 2 g/L |
| Actinase E | 340 kU/L |
| DMSO | 0.2 mL/L |
| DA-67 | 12.6 μmol/L |
| Second reagent | |
| MOPS (pH 7.0) | 50 mmol/L |
| Triton X-405 | 7.1 g/L |
| Peroxidase | 80 kU/L |
| FPOX-CET | 2.5 kU/L |

[Table 3]

| Kit | Halogen oxide | Compound (I) or Compound (II) |
|---|---|---|
| Kit 7 | Potassium bromate (0.6 mmol/L) | 2-Octyl-4-isothiazolin-3-one (2.3 mmol/L) |
| Kit 8 | Potassium bromate (0.6 mmol/L) | 2-Methyl-4-isothiazolin-3-one (4.1 mmol/L) |
| Kit k | Potassium bromate (0.6 mmol/L) | - |
| Kit g | - | 2-Octyl-4-isothiazolin-3-one (2.3 mmol/L) |
| Kit h | - | 2-Methyl-4-isothiazolin-3-one (4.1 mmol/L) |
| Kit i | - | 2-Methylthiazoline (4.9 mmol/L) |
| Kit j | - | - |

Example 4

The kit 1 of Example 1 was used as a kit for measuring HbA1c. Whole blood derived from 16 test subjects suspected of having diabetes mellitus was used as a sample to determine the ratio [HbA1c (%)] of the concentration (amount) of HbA1c to the concentration (amount) of total hemoglobin in each sample by the following procedures:

(1) Preparation of calibration curve for determining total hemoglobin concentration

Nescoat Hemo Kit-N (cyanmethemoglobin method) (manufactured by Alfresa Pharma Corp.), was used as a kit for measuring total hemoglobin. A standard solution (hemoglobin concentration: 15.3 g/dL) included in Nescoat Hemo Kit-N was used as a specimen to prepare a calibration curve showing the relationship between hemoglobin concentration and absorbance.

(2) Preparation of calibration curve for determining HbA1c concentration

For each of two blood cell fractions with HbA1c concentrations valued at 2.97 μmol/L and 7.80 μmol/L respectively, by latex immunoagglutination assay and the total hemoglobin value for each blood cell fraction, the measurement was performed using the kit 1 of Example 1 to determine absorbance for each of the blood cell fractions. Saline was used instead of the blood cell fraction to determine the HbA1c concentration of the saline. The absorbance for the saline was subtracted from the absorbance for each of the blood cell fractions, and the value thus calculated was used as bank-corrected absorbance for the blood cell fraction. A calibration curve showing the relationship between HbA1c concentration (μmol/L) and absorbance was prepared from the blank-corrected absorbance for each of the blood cell fractions and the blank-corrected absorbance (0 Abs) for the saline.

(3) Determination of hemoglobin concentration in each of the blood cell fractions

Each of the samples was centrifuged at 3,000 rpm at 25°C for 5 minutes to obtain a blood cell fraction. For each of the blood cell fractions, the measurement was performed using Nescoat Hemo Kit-N. The hemoglobin concentration (μmol/L) in each of the blood cell fractions was determined from the obtained measurement value and the calibration curve prepared in the paragraph (1).

(4) Determination of HbA1c concentration in each of the blood cell fractions

For each of the blood cell fractions, the measurement was performed using the kit 1 of Example 1. The HbA1c concentration (μmol/L) in each of the blood cell fractions was determined from the obtained measurement value and the calibration curve prepared in the paragraph (2).

(5) Determination of HbA1c (%) (= ratio of HbA1c concentration to hemoglobin concentration)

HbA1c (%) was calculated as a Japan Diabetes Society (JDS) value according to the following equation from the hemoglobin concentration (μmol/L) in each of the blood cell fractions determined in the paragraph (3) and the HbA1c concentration (μmol/L) in each of the blood cell fractions determined in the paragraph (4):

[Equation 1]

$$\text{HbA1c (\%)} = [\text{HbA1c concentration } (\mu\text{mol/L})] / [\text{Hemoglobin concentration } (\mu\text{mol/L})] \times 0.0963 + 1.62$$

(6) Determination of HbA1c (%) in same blood cell fraction by immunoassay

The same blood cell fractions as those used in the determination of HbA1c (%) in the paragraph (5) were used. HbA1c (%) in each of the blood cell fractions was determined by immunoassay using Determiner L HbA1c (manufactured by Kyowa Medex Co., Ltd.) according to the protocol described in the attachment of Determiner L HbA1c.

(7) Correlation between measurement method using measurement kit of the present invention and immunoassay

The correlation between the measurement method of the present invention and the immunoassay was verified from HbA1c (%) determined in the paragraph (5) by the measurement method using the measurement kit of the present invention and HbA1c (%) determined in the paragraph (6) using the immunoassay to determine a correlation coefficient.

The correlation coefficient between the measurement method of the present invention and measurement using Determiner L HbA1c (manufactured by Kyowa Medex Co., Ltd.) was determined in the same way as above except that each of the kits 2 and 3 and the kits a to e of Example 1, the kits 4 to 6 and the kits f to j of Example 2, and the kits 7 and 8 and the kit k of Example 3 was used instead of the kit 1 of Example 1. The results are shown in Table 4.

[Table 4]

Table 4

| Kit | Correlation coefficient |
|---|---|
| Kit 1 | **0.9972** |
| Kit 2 | **0.9969** |
| Kit 3 | **0.9970** |
| Kit a | **0.9985** |
| Kit b | **0.9972** |
| Kit c | **0.9962** |
| Kit d | **0.9957** |

(continued)

| Kit | Correlation coefficient |
|---|---|
| Kit e | **0.9869** |
| Kit 4 | **0.9919** |
| Kit 5 | **0.9889** |
| Kit 6 | **0.9853** |
| Kit f | **0.9987** |
| Kit g | **0.9973** |
| Kit h | **0.9931** |
| Kit i | **0.9914** |
| Kit j | **0.9917** |
| Kit 7 | **0.9919** |
| Kit 8 | **0.9889** |
| Kit k | **0.9935** |

As shown in Table 4, favorable correlation was confirmed between the method for measuring HbA1c using each of the kits of Examples 1 to 3 and the immunoassay. These results demonstrated that HbA1c in a sample could be measured accurately using each of the kits of Examples 1 to 3.

Example 5

Using the kit 1 of Example 1 as a kit for measuring HbA1c, and the samples for confirming an effect of ascorbic acid as prepared by the method mentioned below as samples, a HbA1c concentration (μmol/L) in each of the samples was determined by the following procedures:

(1) Preparation of sample for confirming effect of ascorbic acid

Hemoglobin concentration in a blood cell fraction obtained by a centrifugation of human blood was determined using Nescoat Hemo Kit-N (cyanmethemoglobin method) (manufactured by Alfresa Pharma Corp.), a kit for measuring total hemoglobin. Subsequently, the blood cell fraction was diluted with purified water and hemolyzed to prepare each of the hemoglobin solution having a hemoglobin concentration of 6.4 mg/mL.

Ascorbic acid was added to each of the hemoglobin solutions thus prepared to afford each of the hemoglobin solution having an ascorbic acid concentration of 0.5 mg/dL, 1 mg/dL, 2 mg/dL, or 5 mg/dL, which was used as a sample for confirming an effect of ascorbic acid. In addition, the prepared hemoglobin solution itself was used as a sample for confirming an effect of ascorbic acid having an ascorbic acid concentration of 0 mg/mL.

(2) Preparation of calibration curve for determining HbA1c concentration

A calibration curve showing the relationship between HbA1c concentration (μmol/L) and absorbance was prepared by the method described in the paragraph (2) of Example 4.

(3) Determination of HbA1c concentration in sample for confirming effect of ascorbic acid

Each of the samples for confirming an effect of ascorbic acid as prepared in the paragraph (1) was applied to the measurement using the kit 1 of Example 1 to determine HbA1c concentration (μmol/L) in each of the samples based on the obtained measurement value and the calibration curve prepared in the paragraph (2). When the HbA1c concentration (μmol/L) in the sample for confirming an effect of ascorbic acid having an ascorbic acid concentration of 0 mg/dL was defined as 100, a relative value of the HbA1c concentration (μmol/L) in each of the samples for confirming an effect of ascorbic acid having an ascorbic acid concentration of 0.5 mg/dL, 1 mg/dL, or 2 mg/dL was determined.

A relative value of the HbA1c concentration (μmol/L) in each of the samples was determined by the same procedures of measurement as above except that each of the kits 2 and 3 and the kits a to e of Example 1 was used

instead of the kit 1 of Example 1. The determined relative value is shown in Table 5. A relative value closer to 100 means that the kit is less susceptible to an effect of ascorbic acid.

[Table 5]

Table 5

| Kit | | | HbA1c concentration (relative value) | | | |
|---|---|---|---|---|---|---|
| # | Halogen oxide | Compound (I) or Compound (II) | Ascorbic acid concentration (mg/dL) | | | |
| | | | 0 | 0.5 | 1.0 | 2.0 |
| 1 | Potassium iodate | 2-Octyl-4-isothiazolin-3-one | 100 | 101 | 99 | 94 |
| 2 | Potassium iodate | 2-Methyl-4-isothiazolin-3-one | 100 | 100 | 99 | 99 |
| 3 | Potassium iodate | 2-Methylthiazoline | 100 | 99 | 97 | 84 |
| a | Potassium iodate | - | 100 | 96 | 84 | 66 |
| b | - | 2-Octyl-4-isothiazolin-3-one | 100 | 86 | 68 | 61 |
| c | - | 2-Methyl-4-isothiazolin-3-one | 100 | 90 | 75 | 67 |
| d | - | 2-Methylthiazoline | 100 | 86 | 69 | 61 |
| e | - | - | 100 | 90 | 74 | 63 |

As is evident from Table 5, each of the kits 1 to 3 comprising potassium iodate and compound (I) or compound (II) gave a relative value of HbA1c concentration close to 100 and was thus shown to be less susceptible to an effect of ascorbic acid than the kits free from at least one of potassium iodate and compound (I) or compound (II) (kits a to e).

Example 6

A relative value of the HbA1c concentration (μmol/L) in each of the samples for confirming an effect of ascorbic acid was determined in the same way as in Example 5 except that each of the kits 4 to 6 and the kits f to j of Example 2 was used instead of the kit 1 of Example 1. The determined relative value of the HbA1c concentration (μmol/L) is shown in Table 6.

[Table 6]

Table 6

| Kit | | | HbA1c concentration (relative value) | | | |
|---|---|---|---|---|---|---|
| # | Halogen oxide | Compound (I) or Compound (II) | Ascorbic acid concentration (mg/dL) | | | |
| | | | 0 | 0.5 | 1.0 | 2.0 |
| 4 | Potassium iodate | 2-Octyl-4-isothiazolin-3-one | 100 | 98 | 95 | 86 |
| 5 | Potassium iodate | 2-Methyl-4-isothiazolin-3-one | 100 | 98 | 96 | 95 |
| 6 | Potassium iodate | 2-Methylthiazoline | 100 | 97 | 95 | 84 |
| f | Potassium iodate | - | 100 | 98 | 93 | 76 |
| g | - | 2-Octyl-4-isothiazolin-3-one | 100 | 80 | 66 | 60 |
| h | - | 2-Methyl-4-isothiazolin-3-one | 100 | 93 | 80 | 67 |
| i | - | 2-Methylthiazoline | 100 | 88 | 74 | 59 |
| j | - | - | 100 | 89 | 74 | 63 |

As is evident from Table 6, each of the kits 4 to 6 comprising potassium iodate and compound (I) or compound (II) gave a relative value of HbA1c concentration close to 100 and was thus shown to be less susceptible to an effect of ascorbic acid than the kits free from either or both of potassium iodate and compound (I) or compound (II) (kits f to j).

Example 7

A relative value of the HbA1c concentration (μmol/L) in each of the samples for confirming an effect of ascorbic acid was determined in the same way as in Example 5 except that each of the kits 7 and 8 and the kits k and g to j of Example 3 was used instead of the kit 1 of Example 1. The determined relative value of the HbA1c concentration (μmol/L) is shown in Table 7.

[Table 7]

Table 7

| Kit | | | HbA1c concentration (relative value) | | | |
|---|---|---|---|---|---|---|
| # | Halogen oxide | Compound (I) or Compound (II) | Ascorbic acid concentration (mg/dL) | | | |
| | | | 0 | 0.5 | 1.0 | 2.0 |
| 7 | Potassium bromate | 2-Octyl-4-isothiazolin-3-one | 100 | 98 | 100 | 90 |
| 8 | Potassium bromate | 2-Methyl-4-isothiazolin-3-one | 100 | 96 | 95 | 82 |
| k | Potassium bromate | - | 100 | 97 | 90 | 63 |
| g | - | 2-Octyl-4-isothiazolin-3-one | 100 | 80 | 66 | 60 |
| h | - | 2-Methyl-4-isothiazolin-3-one | 100 | 93 | 80 | 67 |
| i | - | 2-Methylthiazoline | 100 | 88 | 74 | 59 |
| j | - | - | 100 | 89 | 74 | 63 |

As is evident from Table 7, each of the kits 7 and 8 comprising potassium bromate and compound (I) or compound (II) gave a relative value of HbA1c concentration close to 100 and was thus shown to be less susceptible to an effect of ascorbic acid than the kits free from either or both of potassium bromate and compound (I) or compound (II) (kits k and g to j).

These results demonstrated that the effect of ascorbic acid was significantly suppressed in the measurement of HbA1c using the kit comprising a halogen oxide and compound (I) or compound (II).

**Industrial Applicability**

The present invention provides a method for suppressing an effect of ascorbic acid on a measurement of glycated hemoglobin in a glycated hemoglobin-containing sample, and a reagent for measuring glycated hemoglobin in a glycated hemoglobin-containing sample. The method for suppressing an effect of ascorbic acid and the reagent for measuring glycated hemoglobin according to the present invention are useful in the diagnosis or the like of diabetes mellitus.

**Claims**

**1.** A method for suppressing the effects of ascorbic acid on a measurement of glycated hemoglobin in a glycated hemoglobin-containing sample, comprising reacting a proteolytic enzyme with the glycated hemoglobin-containing sample, reacting a generated glycated peptide with a glycated peptide oxidase, and then measuring a generated hydrogen peroxide, wherein the reaction of the glycated peptide with the glycated peptide oxidase is performed in the presence of a halogen oxide and a substance selected from the group consisting of a substance represented by formula (I):

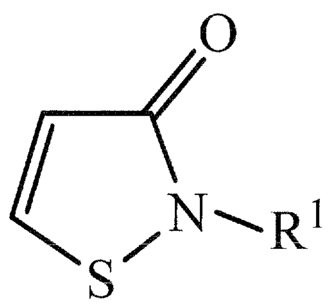

(I)

wherein $R^1$ represents substituted or unsubstituted alkyl, and a substance represented by formula (II):

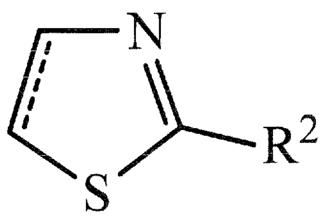

(II)

wherein $R^2$ represents substituted or unsubstituted alkyl; and --- represents a single bond or a double bond.

2. The method according to claim 1, wherein the halogen oxide is a halogen oxide selected from the group consisting of iodic acid or a salt thereof and bromic acid or a salt thereof.

3. The method according to claim 1 or 2, wherein the glycated hemoglobin is hemoglobin A1c.

4. A reagent for measuring glycated hemoglobin in a sample, comprising a proteolytic enzyme, a glycated peptide oxidase, a halogen oxide, a substance selected from the group consisting of a substance represented by formula (I) :

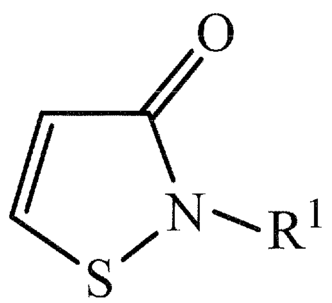

(I)

wherein $R^1$ represents substituted or unsubstituted alkyl, and a substance represented by formula (II):

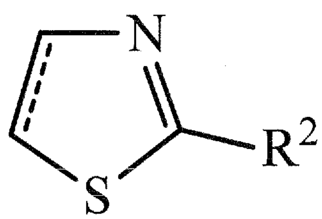

(II)

wherein $R^2$ represents substituted or unsubstituted alkyl; and --- represents a single bond or a double bond, and a reagent for measuring hydrogen peroxide.

5. The reagent according to claim 4, wherein the halogen oxide is a halogen oxide selected from the group consisting of iodic acid or a salt thereof and bromic acid or a salt thereof.

**6.** The reagent according to claim 4 or 5, wherein the glycated hemoglobin is hemoglobin A1c.

**7.** The reagent according to any one of claims 4 to 6, wherein the reagent is intended to suppress an effect of ascorbic acid on the measurement of glycated hemoglobin in the sample.

**INTERNATIONAL SEARCH REPORT**

International application No.
PCT/JP2013/052669

A. CLASSIFICATION OF SUBJECT MATTER
*C12Q1/28*(2006.01)i, *C12Q1/37*(2006.01)i

According to International Patent Classification (IPC) or to both national classification and IPC

B. FIELDS SEARCHED

Minimum documentation searched (classification system followed by classification symbols)
C12Q1/28, C12Q1/37

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2013 |
| Kokai Jitsuyo Shinan Koho | 1971-2013 | Toroku Jitsuyo Shinan Koho | 1994-2013 |

Electronic data base consulted during the international search (name of data base and, where practicable, search terms used)
CAPlus/BIOSIS/MEDLINE/WPIDS(STN), JSTPlus/JMEDPlus/JST7580(JDreamIII)

C. DOCUMENTS CONSIDERED TO BE RELEVANT

| Category* | Citation of document, with indication, where appropriate, of the relevant passages | Relevant to claim No. |
|---|---|---|
| A | JP 2009-544315 A (General Atomics), 17 December 2009 (17.12.2009), & US 2008/0096230 A1 & US 2008/0299597 A1 & EP 2044444 A & WO 2008/013874 A1 | 1-7 |
| A | WO 2003/107011 A1 (Arkray, Inc.), 24 December 2003 (24.12.2003), & JP 2007-147630 A & US 2005/0221415 A1 & EP 1515144 A1 | 1-7 |
| A | JP 08-038195 A (Hitachi Chemical Co., Ltd.), 13 February 1996 (13.02.1996), (Family: none) | 1-7 |
| A | JP 11-124373 A (Sumitomo Chemical Co., Ltd.), 11 May 1999 (11.05.1999), (Family: none) | 1-7 |

☒ Further documents are listed in the continuation of Box C.  ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 09 April, 2013 (09.04.13) | 16 April, 2013 (16.04.13) |

| Name and mailing address of the ISA/ | Authorized officer |
|---|---|
| Japanese Patent Office | |
| Facsimile No. | Telephone No. |

Form PCT/ISA/210 (second sheet) (July 2009)

**INTERNATIONAL SEARCH REPORT**

International application No.
PCT/JP2013/052669

C (Continuation). DOCUMENTS CONSIDERED TO BE RELEVANT

| Category* | Citation of document, with indication, where appropriate, of the relevant passages | Relevant to claim No. |
|---|---|---|
| A | JP 56-151358 A (Boehringer Mannheim GmbH), 24 November 1981 (24.11.1981), & US 4743559 A & EP 37056 A1 & DE 3012368 A | 1-7 |
| P,A | WO 2012/173185 A1 (Kyowa Medex Co., Ltd.), 20 December 2012 (20.12.2012), (Family: none) | 1-7 |
| P,A | WO 2012/020744 A1 (Kyowa Medex Co., Ltd.), 16 February 2012 (16.02.2012), (Family: none) | 1-7 |

Form PCT/ISA/210 (continuation of second sheet) (July 2009)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 56039198 A
- JP 1041223 A
- JP 63067139 A
- JP 63039871 A
- JP 56151358 A
- JP 8271504 A